# EUROPEAN PATENT APPLICATION

(11) **EP 4 840 898 A1**
(43) Date of publication of application: **18.11.2026**
(21) Application number: 25176044.3
(22) Date of filing: 13.05.2025
(51) Int. Cl.: C08J 5/00, C08J 11/06, B29B 17/00, B65D 37/00, A61F 13/00, B29D 7/01

(54) **FLEXIBLE PACKAGES FOR HYGIENE ARTICLE**

(71) Applicant: The Procter & Gamble Company, Cincinnati, OH 45202 (US)
(72) Inventor: KOHLWEYER, Christian, 65824 Schwalbach am Taunus (DE)
(74) Representative: P&G Patent Germany

(57) **Abstract**

Flexible packages made of polymeric film for hygiene articles, wherein the flexible package comprises post-consumer recycled (PCR) polymeric material derived from recycling of a laminate comprising a paper layer, a barrier layer and a polymer layer. Use of such PCR material for and method of making flexible packages made of polymeric film for hygiene articles.

## Description

### FIELD

The present disclosure relates to flexible packages made of polymeric film for hygiene articles, the use of post-consumer recycled material in flexible packages made of polymeric film for hygiene articles as well as methods for making such flexible packages.

### BACKGROUND

Providing hygiene articles in flexible packages made of polymeric film material is widely known. For example, absorbent articles, such as diapers, bibs, wipes, sanitary napkins, tampons, etc. are commonly packaged in flexible packages made of polymeric film. Polymeric film, plastic film respectively, is preferred as the primary package of many hygiene articles because plastic film may withstand the rigors of a packaging process, given a plastic film's ability to flex and stretch. Also, because polymeric film can flex and stretch without tearing upon the application of force, opening features disposed in plastic packages tend to form clean openings while preserving the integrity of the rest of the package. In addition, polymeric films can also hygiene articles from moisture and other contaminants during shipping and prior to use.

Consumer demand for products and packaging made at least partially from renewable and/or recycled resources has increased significantly over the past decade and has become a driver of innovation for new and improved hygiene articles and packaging materials. In the context of polymeric film-based packaging, one way to address this consumer demand is to replace at least a proportion of new petrol-based plastic material with plastic waste material, also known as recycled plastic material.

However, adding recycled materials back into the manufacturing process often negatively impacts the quality of the resulting new flexible film packaging. These adverse effects can relate to a number of properties, such as mechanical aspects (e.g. tensile strength or elongation at break), optical properties of the film material, or reduced processability, such as printing or sealing. Due to these problems, flexible film packages often comprise only relatively small amounts of recycled materials.

In particular, post-consumer recycled materials may be contaminated with foreign matter and trace chemicals. It would thus be desirable to provide flexible packages in which the above-mentioned negative effects are reduced or even off-set while at the same time enabling relatively high amounts of post-consumer recycled materials in the packaging material.

### SUMMARY

The invention relates to a flexible package for hygiene articles. The flexible package are made of made of polymeric film. The polymeric film and thus the packages comprise post-consumer recycled (PCR) polymeric material derived from recycling of a laminate comprising a paper layer, a barrier layer and a polymer layer. In particular, the PCR polymeric material may be derived from post-consumer recycling of paper composite cartons, in particular paper composite cartons used for beverages or other liquid consumer goods.

The invention further relates to the use of post-consumer recycled (PCR) material for making flexible package made of polymeric film for hygiene articles. The PCR polymeric material is derived from recycling of a laminate comprising a paper layer, a metal barrier layer and a polymer layer, in particular from recycling of a laminate comprising a paper layer, a metal barrier layer and a polymer layer comprised in paper composite cartons, in particular paper composite cartons used for beverages or other liquid consumer goods.

The invention further relates to a method of making a flexible package made of polymeric film for hygiene articles. The method comprises the steps of
a) obtaining post-consumer recycled (PCR) polymeric material from post-consumer recycling of a laminate comprising a paper layer, a metal barrier layer and a polymer layer;
b) providing the PCR polymeric material obtained in step a);
c) melting the PCR polymeric material and optionally mixing it with further polymeric material different from the PCR polymeric material;
d) extruding the molten polymeric material to form a polymeric film;
e) providing print on one or both surfaces of the polymeric film; and
f) converting the polymeric film into the flexible package.

### BRIEF DESCRIPTION OF THE DRAWINGS

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:
Fig. 1 is a schematic representation of a polymeric film;
Fig. 2 is a schematic representation of a polymeric film comprising printed graphics disposed on a first surface of the polymeric film;
Fig. 3 is a schematic representation of a polymeric film comprising printed graphics disposed on a second surface of the polymeric film;
Fig. 4 is a schematic representation of a multilayer polymeric film comprising two layers;
Fig. 5 is a schematic representation of a multilayer polymeric film comprising three layers;
Fig. 6 is a schematic representation of a package formed at least partially from a polymeric film;
Fig. 7 is a schematic representation of a package formed at least partially from a polymeric film having a handle and an opening feature;
Fig. 8 is a schematic representation showing a package constructed with a flow wrap process;
Fig. 9 is a cross-sectional view of the package showing hygiene articles disposed therein;
Fig. 10 is a plan view of an example hygiene article in the form of a diaper, garment-facing surface facing the viewer, in a flat laid-out state;
Fig. 11 is a front perspective view of a hygiene article in the form of a pant;
Fig. 12 is a plan view of an absorbent article in the form of a sanitary napkin.

### DETAILED DESCRIPTION

The following definitions may be useful in understanding the present disclosure.

"Machine direction" (MD) is used herein to refer to the direction of material flow through a process. In addition, relative placement and movement of material can be described as flowing in the machine direction through a process from upstream in the process to downstream in the process.

"Cross direction" (CD) is used herein to refer to a direction that is not parallel with, and usually perpendicular to, the machine direction.

"Sealed" refers herein to a package having an interior that is inaccessible until the package is ruptured. It also refers to two or more layers of a polymeric film which are overlaying each other and are joined to each other, e.g. to form side seams of a flexible package. In the side seam, the layers of polymeric film are sealed such that they cannot be separated without rupturing or tearing the polymeric film.

"Virgin polymeric material" as used herein, means the polymeric material produced directly from the petrochemical or plant based feed-stock, such as natural gas or crude oil or sugar cane, which has never been used or processed before.

As used herein, "polymeric resin" refers to polymeric material in any form other than the form of a polymeric film of the present disclosure, including pellets, molten form, precursor scrap film, and the like.

"Resin pellets", as used herein, are small granules of polymeric material, which may have the shape of a cylinder or a disc. The size of the resin pellets may range from 2mm to 5mm with a diameter of a few mm. These resin pellets are molten and formed into the polymeric film of the present invention.

As used herein, "gel" and "gels" refer to any small defect that distorts a film product. Gels may originate from a variety of different sources, including oxidized polymeric materials, crosslinked polymeric materials, highly-entangled polymeric material, solid and/or unmelted resin, and contaminants (such as metal, dirt, wood, or other fragments).

As used herein, "high density polyethylene" (HDPE) means a type of polyethylene defined by a density equal to or greater than 0.941g/cm³.

As used herein, "low density polyethylene" (LDPE) means a type of polyethylene defined by a density equal to or less than 0.925 g/cm³.

As used herein, "medium density polyethylene" (MDPE) means a type of polyethylene defined by a density range of 0.926-0.940 g/cm³.

As used herein, "linear low density polyethylene" (LLDPE) means a type of Low Density Polyethylene characterized by substantially linear polyethylene, with significant numbers of short branches, commonly made by copolymerization of ethylene with longer-chain olefins. Linear low-density polyethylene differs structurally from conventional low-density polyethylene (LDPE) because of the absence of long chain branching. The linearity of LLDPE results from the different manufacturing processes of LLDPE and LDPE. In general, LLDPE is produced at lower temperatures and pressures by copolymerization of ethylene and such higher alpha-olefins as butene, hexene, or octene. The copolymerization process produces a LLDPE polymer that has a narrower molecular weight distribution than conventional LDPE and in combination with the linear structure, significantly different rheological properties.

As used herein, "hygiene product" means diapers, pants, wet and dry wipes, tampons, adult incontinence products, paper towels, toilet tissue and facial tissue.

As used herein, "absorbent article" refers to devices that absorb and contain body exudates, and, more specifically, refers to devices that are placed against or in proximity to the body of the wearer to absorb and contain the various exudates discharged from the body. Absorbent articles may include diapers (baby diapers and diapers for adult incontinence), pants (for babies or for adults), absorbent inserts (which are intended to be inserted into an outer cover to form a diaper or pant), feminine care absorbent articles such as sanitary napkins or pantiliners, and the like. As used herein, the term "exudates" includes, but is not limited to, urine, blood, vaginal discharges, sweat and fecal matter. Preferred absorbent articles of the present invention are disposable absorbent articles, more preferably disposable diapers and disposable pants.

As used herein, "disposable" is used in its ordinary sense to mean an article that is disposed or discarded after a limited number of usages over varying lengths of time, for example, less than 20 usages, less than 10 usages, less than 5 usages, or less than 2 usages. If the disposable absorbent article is a diaper, a pant, sanitary napkin, sanitary pad or wet wipe for personal hygiene use, the disposable absorbent article is most often intended to be disposed after single use.

As used herein, " laminate to be recycled " refers to the laminate which is subject to the recycling process. As such the laminate to be recycled is isolated from post-consumer waste. The laminate to be recycled may form part of paper composite cartons.

### Recycled material and laminate to be recycled

Recycling of polymeric materials for making new products or materials, such as packaging materials, is widely known. Generally, it is distinguished between pre-consumer/ post-industrial recycled material (also referred to as PIR) and post-consumer recycled material (also referred to as PCR), e.g. as described in ISO norm 14021:2016 (E).

PIR relates to reintroduction of waste generated in a manufacturing process back into the original manufacturing process.

In contrast to PIR materials, PCR material is waste created by consumers after a product has reached the end of its use. This material is diverted from the landfill or recuperated in recollection systems and utilized in the production of other commodities. PCR material is exposed to potential contamination sources both during use and during the recycling process, thus typically PCR materials exhibit higher levels of contamination in comparison to PIR materials. Additionally, it is harder to derive polymeric materials containing only one sort of plastic, such as polyethylene (PE), polypropylene (PP) or polyesters such as polyethylene terephthalate (PET) or even one sort of plastic with defined properties such as LDPE and HDPE.

For producing polymeric films for packaging from PCR materials, such contaminations negatively impact both processability, in particular during film blowing, and mechanical and/ or optical properties of the final film. This challenge is further enhanced for packaging of hygiene articles: in addition to contaminants impacting the processibility and mechanical and/ or optical properties, contaminants can transfer from the packaging to the enclosed articles. Hence, PCR material - and films and packaging derived from it - have to be free or contain minimal amounts of contaminants and/ or trace chemicals. Hence, a high-quality PCR polymeric material is required for making flexible packages made of polymeric film 100 for hygiene articles 1004.

Flexible package made of polymeric film 100 for hygiene articles 1004 are provided. The flexible package comprises post-consumer recycled (PCR) polymeric material derived from recycling of a laminate comprising a paper layer, a barrier layer and a polymer layer. It has been found that the PCR polymeric materials derived from such a laminate contains low amounts of contaminants. Without being bound to theory it is envisioned that the polymeric layer is at least partially protected from contamination by other PCR waste by the at least two other layers comprised by the laminate. By choosing such a laminate as source of the PCR material, also a more targeted sorting of the waste to be recycled is enabled.

In the laminate to be recycled, the barrier layer may constitute the middle layer, while the paper layer and the polymeric layer may constitute the outer layers.

The barrier layer comprised by the laminate to be recycled may comprise metal. The barrier layer comprised by the laminate to be recycled may comprise aluminum. Preferably the barrier layer comprised by the laminate to be recycled may be an aluminum barrier layer. Although the recycling includes steps to separate the layers of the laminate to be recycled, the PCR polymeric material may still contain traces of paper material and/ or barrier material. The PCR polymeric material may comprise from 1 µg to 1500 µg, preferably from 5 µg to 1000 µg, more preferably from 10 µg to 800 µg, even more preferably from 100 µg to 600 µg, even more preferably from 200 µg to 500 µg, or even from 300 µg to 400 µg metal per 1 g of the PCR polymeric material from 0.0001 mg to 10 mg metal per 1 g of polymeric film 100. The PCR polymeric material may comprise from 1 µg to 1500 µg, preferably from 5 µg to 1000 µg, more preferably from 10 µg to 800 µg, even more preferably from 100 µg to 600 µg, even more preferably from 200 µg to 500 µg, or even from 300 µg to 400 µg aluminum per 1 g of the PCR polymeric material. It has been found that the PCR polymeric material is processable in the extrusion of the polymeric film 100 despite comprising traces of metals, in particular aluminum.

The polymeric layer comprised by the laminate to be recycled may comprise a polyolefin. Preferably the polymeric layer comprised by the laminate to be recycled may comprise polypropylene (PP), polyethylene (PE) and/ or PE-PP copolymers, more preferably LDPE, HDPE, LLDPE or mixtures thereof. In particular, the polymeric layer comprised by the laminate to be recycled may comprise PE, more particular LDPE. The polymeric layer comprised by the laminate to be recycled may be a polyolefin. Preferably the polymeric layer comprised by the laminate to be recycled may polypropylene (PP), polyethylene (PE) and/ or PE-PP copolymers, more preferably LDPE, HDPE, LLDPE or mixtures thereof. In particular, the polymeric layer comprised by the laminate to be recycled may be PE, more particular LDPE. The polymeric layer comprised by the laminate to be recycled may comprise a food-grade polymer or may be made of a food-grade polymer. Food grade polymers are food safe plastics that can be used without affecting consumer health. FDA Compliance refers to the Food and Drug Administration, which sets standards for the safe storage of food and beverage besides drugs.

The PCR polymeric material may be derived from post-consumer recycling of paper composite cartons. In other words, the PCR polymeric material may be derived from post-consumer recycling a laminate comprising a paper layer, a barrier layer and a polymer layer forming part of paper composite cartons. In paper composite cartons, the polymeric layer typically constitutes the layer facing the inside of the carton, while the paper layer faces the outside, with the barrier layer being located in between the polymeric layer and the paper layer. During the collection and sorting of the waste, the carton typically remains in its closed form. Thus, contamination of the polymeric layer by other waste during collection and sorting of the post-consumer waste can be minimized.

In the laminate to be recycled comprised by paper composite cartons, the barrier layer may constitute the middle layer, while the paper layer and the polymeric layer may constitute the outer layers. In particular, the paper composite cartons may be intended to enclose a hygiene article 1004. The polymeric layer may face the inside of the paper composite cartons. Thus, the polymeric layer may be in contact with the hygiene article 1004 prior, during and potentially after consumer use of the paper composite cartons. The paper layer may face the outside of the paper composite cartons. Thus, the paper layer may not be in contact with the hygiene article 1004 prior, during and potentially after consumer use of the paper composite cartons but may be in contact with the other waste during waste collection and sorting.

In particular, the PCR polymeric material may be derived from post-consumer recycling of paper composite cartons used for beverages or other liquid consumer goods, preferably for beverages. Paper composite cartons, which comprised beverages or other liquid consumer goods, may be more readily cleaned prior to recycling/ purification steps. Further, the contained goods typically do not comprise contaminants that may be transferred to the polymeric inner layer of the composite. In addition, paper composite cartons used for beverages or other liquid consumer goods, already have to meet high requirements regarding comprised contaminants, in particular for the polymeric layer being in direct contact with the liquids. In particular for beverages, food-grade polymeric materials have to be employed.

Due to the reasons already discussed, PCR polymeric materials derived from the laminate to be recycled may contain low amounts of contaminants. The PCR polymeric material may meet one or more of the criteria a) to f) by comprising less than
a) 200 ng/kg, preferably less than 100 ng/kg of 3,3',4,4'-Tetrachloro-l,l'-biphenyl (PCB 77);
b) 2000 ng/kg, preferably less than 1000 ng/kg of 2,3',4,4',5-Pentachloro-l, l '-biphenyl (PCB 118);
c) 150 ng/kg, preferably less than 100 ng/kg of l,2,3,4,6,7,8,9-Octachlorodibenzo[b,e][l,4]dioxin (OCDD);
d) 10.0 ng/kg, preferably less than 5.0 ng/kg of 1,2,3,4,6, 7,8,9-Octachlorodi benzofuran (OCDF);
e) 500 µg/kg, preferably less than 400 µg/kg of bisphenol A; and/ or
f) 25,000 µg/kg, preferably less than 20,000 µg/kg of diisononyl phthalate; according to the Trace Chemicals Test Method described herein.
Preferably, the PCR polymeric material may meet two or more, preferably three or more, more preferably four or more and most preferably all of the criteria a) to f).

The PCR polymeric material may be derived from mechanical recycling. Mechanical recycling is the process of recovering polymeric waste by mechanical processes such as sorting, washing, drying, grinding, re-granulating and compounding. Mechanical recycling does not change the chemical structure of the material.

### Polymeric film and flexible package made of polymeric film

The flexible package can take any shape and size known in the art and suitable for the product to be contained therein. For example, flexible packages may include opposing first and second panels. Each panel may define a top edge portion, a bottom edge portion, a left side edge portion, and a right side edge portion. The first and second panels may be joined at a first side seam along left side edge portions of the first and second panels and at a second side seam along right side edge portions of the first and second panels. The flexible package may include an opening feature 700 along a top or bottom edge portion of the package for opening the flexible package. The opening feature may, for example be a perforation. The opening feature may be reclosable. The reclosable features may include a lid, tape tab fastener, hook and loop fastener, snap, button, or latch, for example. The flexible package may also comprise a handle for ease of carrying.

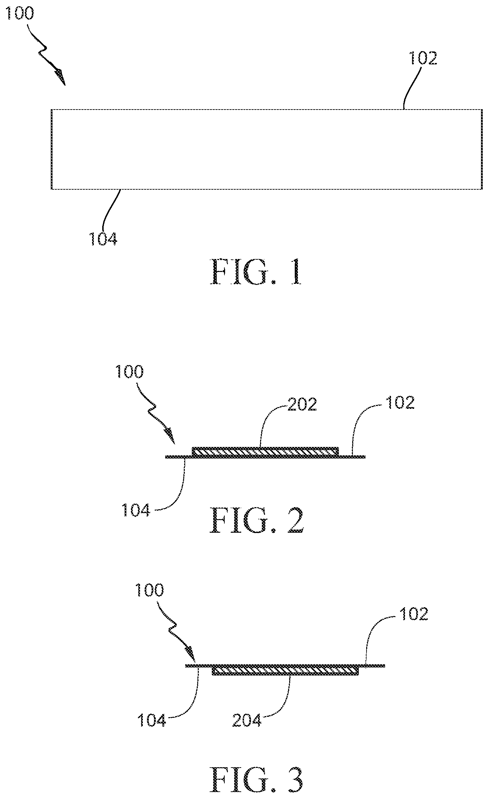

Referring to Fig. 1, the polymeric film 100 may comprise a first surface 102 and a second surface 104. For purposes of balancing economy of material usage and maximization of tensile strength of the film, the polymeric film 100 may have a thickness (caliper) of from about 20 µm to about 100 µm, from about 25 µm to about 98 µm, or from about 28 µm to about 95 µm, specifically reciting every 1.0 µm increment within these ranges and all ranges formed therein or thereby. The polymeric film 100 of the present disclosure is a flexible film. As used herein, the term "flexible" refers to a relatively thin, easily deformable material, as opposed to a "rigid" material that exhibits resistance to deformation. The polymeric film 100 of the present disclosure may not be a rigid film.

The first surface 102 of the polymeric film 100 may comprise a printed graphic 202, as shown in Fig. 2, or may be free of a printed graphic. The second surface 104 may comprise a printed graphic 204, as shown in Fig. 3, or may be free of a printed graphic. A "graphic" may include the depiction of a design or designs, any recognizable indicia such as a number, a letter, a word, a brand name, an icon, a logo, a character, any shape and/or symbol (for example hearts, clouds, animals, etc.), as well as a full flood of pigment across the surface, or a portion of the surface, of the polymeric film 100. In a form, the first surface 102 may comprise a printed graphic and the second surface 104 may be devoid of a printed graphic. In another form, the first surface 102 may be devoid of a printed graphic and the second surface 104 may comprise a printed graphic. The printed graphic may be formed by ink or any other pigmented medium known in the art.

The polymeric film 100 may be a monolayer material, such that the film, throughout its thickness, consists of the same material composition. In one example, the polymeric film 100 may be a monolayer material and the package may comprise diapers and/ or pants. Alternatively, the polymeric film 100 may comprise more than one layer, such a first outer layer, a second outer layer and one or more inner layers. The first outer layer may face towards the interior of the flexible package, with first areas of the first outer layer being sealed to second areas of the first outer layer to form seams (e.g. side seams). The second outer layer may comprise a printed surface area.

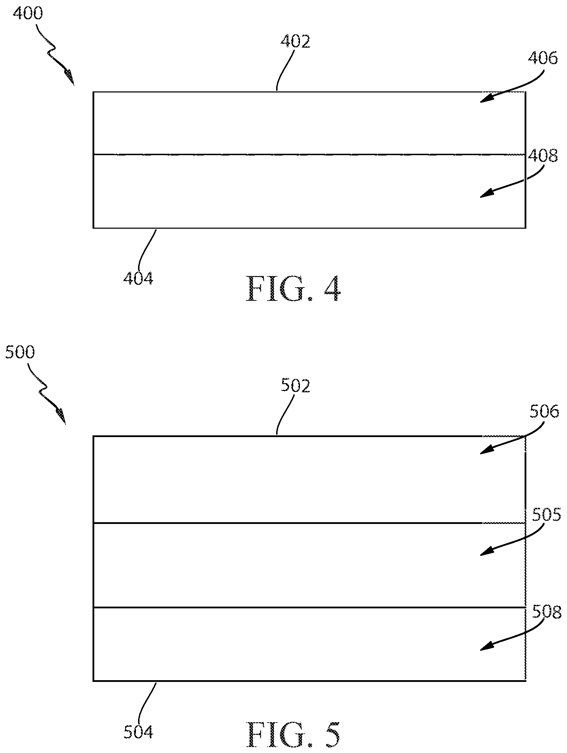

Referring to Figs. 4 and 5, the polymeric films of the present disclosure may be formed from two or more layers of polymeric material. As shown in Fig. 4, the polymeric film 400 comprises a first film layer 406 and a second film layer 408. The first film layer 406 may form the first surface 402 of the polymeric film 400. The second film layer 408 may form the second surface 404 of the polymeric film 400. As shown in Fig. 5, an intermediate film layer 505 may be disposed between a first film layer 506 and a second layer 508. The first film layer 506 may form the first surface 502, and the second film layer 508 may form the second surface 504.

The different layers of the multilayer films of the present disclosure may differ in thickness (caliper). Referring to Fig. 5, the intermediate film layer 505 may have a greater thickness than the first film layer 506 and/or the second film layer 508. The thickness of the first film layer 506 and/or the second film layer 508 may be approximately one-half the thickness of the intermediate film layer 505. In a form, the thicknesses of the different layers of the multilayer films may be in a ratio of 1 : 2 : 1 (first film layer : intermediate film layer : second film layer). In another form, the thicknesses of the different layers of the multilayer films may be in a ratio of 0.4 : 2 : 0.4 (first film layer : intermediate film layer : second film layer). In yet another form, the thicknesses of the different layers of the multilayer films may be in a ratio of 0.5 : 2 : 1 (first film layer : intermediate film layer : second film layer).

The first outer layer may therefore comprise small quantities of additives which improve the seal ability of the first outer layer. Likewise, the second outer layer may comprise small quantities of additives which improve the printability of the second outer layer.

The polymeric film 100 may be a laminate comprising at least one polymeric layer and optionally a barrier layer. In one example, the polymeric film 100 may be a laminate comprising at least one polymeric layer and optionally a barrier layer and the package may comprise wipes.

Prior to being converted into a flexible package, the polymeric film 100 may be provided with print on one or both of its surfaces. The polymeric film 100 may be provided with print at least on the outer surface, i.e. on the surface which forms the exterior of the flexible package.

The one or more inner layers may contribute to the overall durability and strength (such as the tensile strength) of the polymeric film 100.

The one or more inner layers may have a higher basis weight than the basis weight of each of the first and second outer layers. The one or more inner layers may have a higher basis weight that the basis weight of the first and second outer layers combined. If the polymeric film 100 has more than one inner layer, the foregoing relates to the combined basis weight of the all inner layers.

If the polymeric film 100 comprises more than one layer, the individual layers may comprise different percentages of the PCR material derived from recycling of a laminate comprising a paper layer, a barrier layer and a polymer layer.

The polymer layer of the laminate to be recycled may be "substantially" the same as the polymeric film 100 of the flexible package, as the polymeric composition in each of the respective layers of the polymeric film 100 may likely not be fully identical with the polymer layer of the laminate to be recycled. As a consequence, additives or the like, which were present in the polymer layer of the laminate to be recycled, may be present in certain layers of the polymeric film 100 if such layer comprises recycled material from the polymer layer of the laminate to be recycled. The term "substantially" is used herein to take such minor deviations into account.

The polymeric film may comprise a polyolefin. Preferably the polymeric film may comprise polypropylene (PP), polyethylene (PE) and/ or PE-PP copolymers, more preferably LDPE, HDPE, LLDPE or mixtures thereof. In particular, the polymeric film may comprise PE, more particular LDPE. The polymeric film may consist of a polyolefin and additives. Preferably the polymeric film may consist of polypropylene (PP), polyethylene (PE) and/ or PE-PP copolymers and additives, more preferably LDPE, HDPE, LLDPE or mixtures thereof and additives. In particular, the polymeric film may consist of PE and additives, more particular LDPE and additives.

So far, recycling of materials often leads to a quality decrease in the products comprising the recycled materials. Hence, PCR and also PIR materials are typically only added at relative low levels. It has now been found that the PCR polymeric materials derived from the laminate to be recycled comprising a paper layer, a barrier layer and a polymer layer according to the present invention can be added at relatively high levels to flexible packages made of polymeric film 100 without substantially compromising key mechanical properties. The inventors have proven that polymeric films comprising the PCR polymeric material results in polymeric films with good tensile strength and elongation at break. The inventors have measured these properties in the polymeric films comprising the PCR material and have also compared them to the mechanical properties from polymeric films not comprising any recycled materials (i.e. they are only formed of so-called virgin material). The results of the measurements are set out below.

The polymeric film 100 may comprise at least 5 weight-%, preferably at least 10 weight-%, more preferably at least 15 weight-%, even more preferably at least 20 weight%, even more preferably at least 25 weight-% or even at least 35%weight-% of PCR polymeric material based on the total weight of the polymeric film 100. The polymeric film 100 may comprise at least 5 weight-%, preferably at least 10 weight-%, more preferably at least 15 weight-%, even more preferably at least 20 weight-%, even more preferably at least 25 weight-% or even at least 35 weight-% of PCR polymeric material derived from recycling of a laminate comprising a paper layer, a barrier layer and a polymer layer. The weight-% are based on the total weight of the polymeric film 100.

The polymeric film 100 may additionally comprise post-industrial recycled (PIR) polymeric material. The polymeric film 100 may comprise at least 30 weight-%, preferably at least 40 weight-%, more preferably at least 50 weight-%, even more preferably at least 60 weight-% or even at least 80% of PIR polymeric material based on the total weight of the polymeric film 100. The polymeric film 100 may comprise at least 50 weight-%, preferably at least 60 weight-%, more preferably at least 70 weight-%, even more preferably at least 80 weight-% or even at least 90% of recycled polymeric material based on the total weight of the polymeric film 100. For clarity, recycled material includes PIR material and PCR material. The polymeric film 100 may consist of recycled material and additives. Preferably, the polymeric film 100 may consist of PIR material, PCR material derived from recycling of a laminate comprising a paper layer, a barrier layer and a polymer layer, and additives.

Non-limiting examples of classes of additives include antioxidants, colorants, nanoparticles, antistatic agents, processing aids, slip agents such as Eurucamides or waxes, flame retardants, fillers, and combinations thereof. Fillers exemplarily comprise
- glass including fibers, spheres, hollow spheres, flakes, MgO, SiO₂, Sb₂O₃ or Al₂O₃ glasses;
- inorganic hydroxides such as Al(OH)₃, Mg(OH)₂;
- silicates such as talc, mica, kaolin, wollastonite, montmorillonite, nanoclays, feldspar;
- organics and
- carbon such as graphite, carbon fibers, graphite fibers and flakes, carbon nanotubes, carbon black.

Colorants may be pigments and/or opacifiers. Opacifiers may be selected from TiO₂, CaCO₃, Carbon black, ZnO₂, BaSO₄, and organic dyes. In particular, TiO₂ may be used as additive. TiO₂ may provide the flexible package body and/ or the fitment with an opaque and/ or white appearance.

Accordingly, the polymeric film 100 may comprise at most 90 weight-%, preferably at most 70 weight-%, more preferably at most 50 weight-%, even more preferably at most 20 weight-%, even more preferably at most 15 weight-% or even preferably at most 10 weight-% of virgin polymeric material based on the total weight of the polymeric film 100 or may even be devoid of virgin polymeric material.

The barrier layer comprised by the laminate to be recycled may comprise metal. The barrier layer comprised by the laminate to be recycled may comprise aluminum. Preferably the barrier layer comprised by the laminate to be recycled may be an aluminum barrier layer. The polymeric film 100 may comprise from 1 µg to 1500 µg, preferably from 5 µg to 1000 µg, more preferably from 10 µg to 800 µg, even more preferably from 15 µg to 500 µg, even more preferably from 20 µg to 300 µg, or even from 25 µg to 100 µg metal per 1 g of the polymeric film 100. The polymeric film 100 may comprise from 1 µg to 1500 µg, preferably from 5 µg to 1000 µg, more preferably from 10 µg to 800 µg, even more preferably from 15 µg to 500 µg, even more preferably from 20 µg to 300 µg, or even from 25 µg to 100 µg aluminum per 1 g of the polymeric film 100. It has been found that the PCR polymeric material is processable in the extrusion of the polymeric film 100 despite comprising traces of metals, in particular aluminum, which are then present in the polymeric film 100 as well.

The melt flow rate of the PCR polymeric material may be from 0.2 to 6.0 g/ 10 min, preferably from 0.3 to 5.5 g/ 10 min, more preferably from 0.4 to 5.0 g/ 10 min, even more preferably from 0.5 to 4.8 g/ 10 min or even from 0.8 to 4.6 g/ 10 min according to DIN EN ISO 1133-1 - 2022-10. The melt flow rate of the PCR polymeric material may be from 0.2 to 3.0 g/ 10 min, preferably from 0.5 to 2.5 g/ 10 min, more preferably from 0.8 to 2.3 g/ 10 min or even more preferably from 1.0 to 2.0 g/ 10 according to DIN EN ISO 1133-1 - 2022-10. Alternatively, the melt flow rate of the PCR polymeric material may be from 3.2 to 6.0 g/ 10 min, preferably from 3.5 to 5.5 g/ 10 min, more preferably from 3.8 to 5.3 g/ 10 min or even more preferably from 4.0 to 5.0 g/ 10 according to DIN EN ISO 1133-1 - 2022-10.

Due to the reasons already discussed, PCR polymeric materials derived from the laminate to be recycled and thus the polymeric film 100 may contain low amounts of contaminants. The polymeric film 100 may meet one or more of the criteria a) to f) by comprising less than
a) 40 ng/kg, preferably less than 10 ng/kg of 3,3',4,4'-Tetrachloro-l,l'-biphenyl (PCB 77);
b) 400 ng/kg, preferably less than 100 ng/kg of 2,3',4,4',5-Pentachloro-l, l '-biphenyl (PCB 118);
c) 30 ng/kg, preferably less than 10 ng/kg of l,2,3,4,6,7,8,9-Octachlorodibenzo[b,e][l,4]dioxin (OCDD);
d) 2.0 ng/kg, preferably less than 0.5 ng/kg of 1,2,3,4,6, 7,8,9-Octachlorodi benzofuran (OCDF);
e) 100 µg/kg, preferably less than 40 µg/kg of bisphenol A;
   and/ or
f) 5,000 µg/kg, preferably less than 2,000 µg/kg of diisononyl phthalate; according to the Trace Chemicals Test Method described herein.

Preferably, the polymeric film 100 may meet two or more, preferably three or more, more preferably four or more and most preferably all of the criteria a) to f).

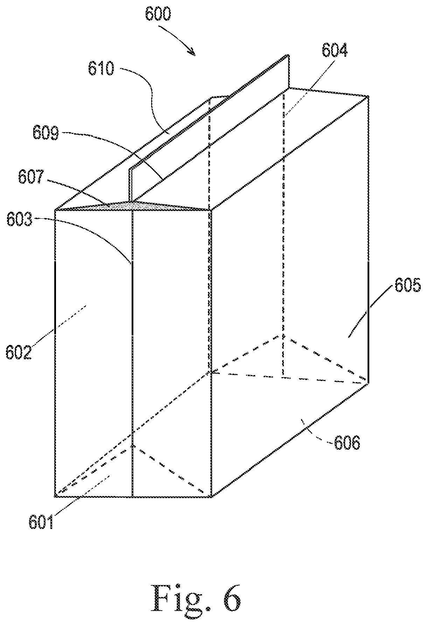

The packages of the present disclosure may be formed from a single sheet of polymeric film 100 of the present disclosure suitably folded to form a bag structure, as shown in Fig. 6. A bag structure 600 may be formed from a single sheet of polymeric film that is suitably folded to form bag gussets 601, 602 and then joined along portions by bonding to form two side seams 603, 604 on opposite sides, to form bag structure 600 with no seam on a first package surface 605, and open at the other end (e.g., a pre-made wicket bag with a top or side gusset). Thereafter, the bag structure may be filled by inserting the hygiene articles 1004 through the open end. The open end opposite first package surface 606 may then be closed by suitably folding to form closing gussets 607, 608, bringing the polymeric film edges together, and bonding them together to form end seam 609 and second package surface 610.

The flexible packages may be wicketed flexible packages. Wicketed flexible packages comprise portions (namely a "wicket panel"), which are removed, typically after the flexible package has been filled with products.

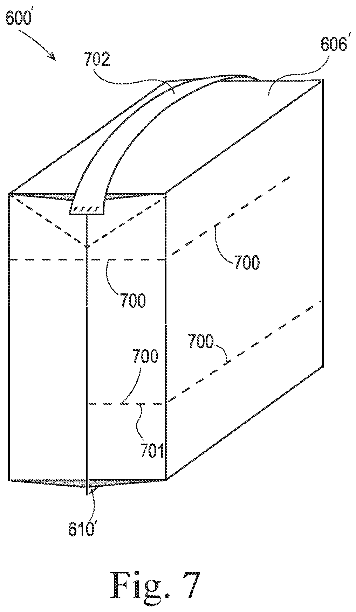

Referring to Fig. 7, the packages 600 may include an opening feature 700 along a top or bottom edge portion of the package for opening the package 600. The opening feature 700 may, for example, be a perforation or line of perforations 701. The opening feature 700 may be reclosable. The reclosable features may include a lid, tape tab fastener, hook and loop fastener, snap, button, or latch, for example. The package 600 may also comprise a handle 702 for ease of carrying.

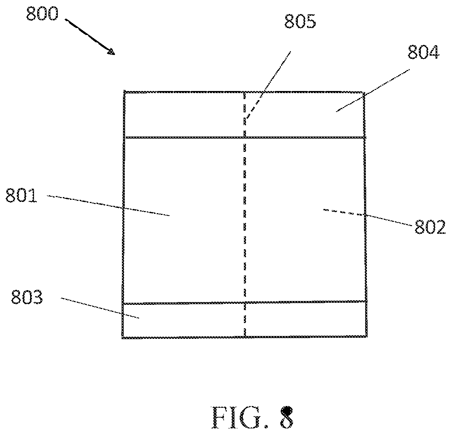

Other package shapes are contemplated, including flow wrap or horizontal form fill-and-seal wrap comprising polymeric films of the present disclosure. Referring to Fig. 8, a flow wrap package 800 may comprise a first surface 801 and an opposing second surface 802. Rounded edges may be provided as a transition between the first surface 801 and the second surface 802. One or more fold lines may be provided between the first surface 801 and the second surface 802. The flow wrap package 800 may further comprise end seals 803 and 804, and a longitudinal seam 805 which may be disposed on the second surface 802. Flow wrap packages may be useful, particularly where a low number of products are included within a package.

Bonds forming any or all of the package seals, such as seams, may be created by welding. (Herein, "weld" refers to a union between separate portions of polymeric film, effected by application of direct or indirect (e.g., ultrasonic) heating energy and pressure that causes separate portions of the film to at least partially melt and fuse together to some extent, forming a bonded area, joint, or seam which cannot be separated without substantial destruction to the remainder of one or both joined portions.) If package-forming machinery forms welds in the polymeric film that join the film stock to itself by applying heating energy that causes the film to fuse to itself, it may be desirable that the film stock be multilayer film, and that the layer(s) to be brought into contact and fused be formed of polymer(s) that have lower melting temperature(s) than those of the polymer(s) used to form the other layer(s). This enables heating energy to be applied to a degree sufficient to heat the layer(s) in contact and cause them to fuse, but not sufficient to cause undesired melting and deformation of the other layer(s), which could cause the package to be misshapen and/or displace and/or distort printing on the film stock.

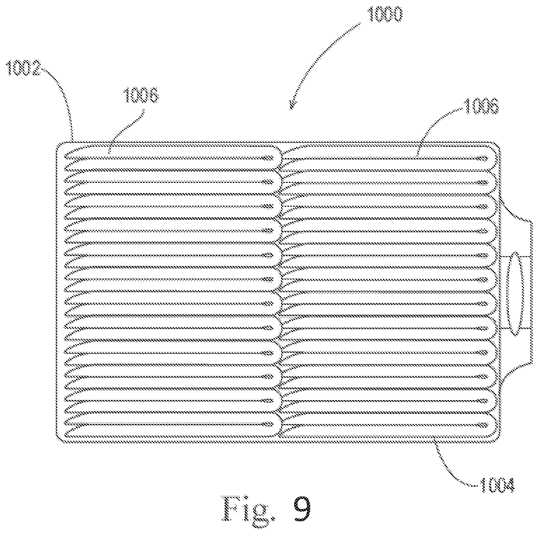

The packages of the present disclosure may comprise a polymeric film of the present disclosure and a hygiene article 1004. As shown in Fig. 9, the packages 1000 may define an interior compartment 1002 such that the polymeric film may at least partially enclose one or more than one hygiene article 1004. The polymeric film may be disposed around and fully enclose the hygiene article 1004. The hygiene article 1004 may be disposed within the polymeric film. In a form, the packages of the present disclosure consist of the polymeric films of the present disclosure.

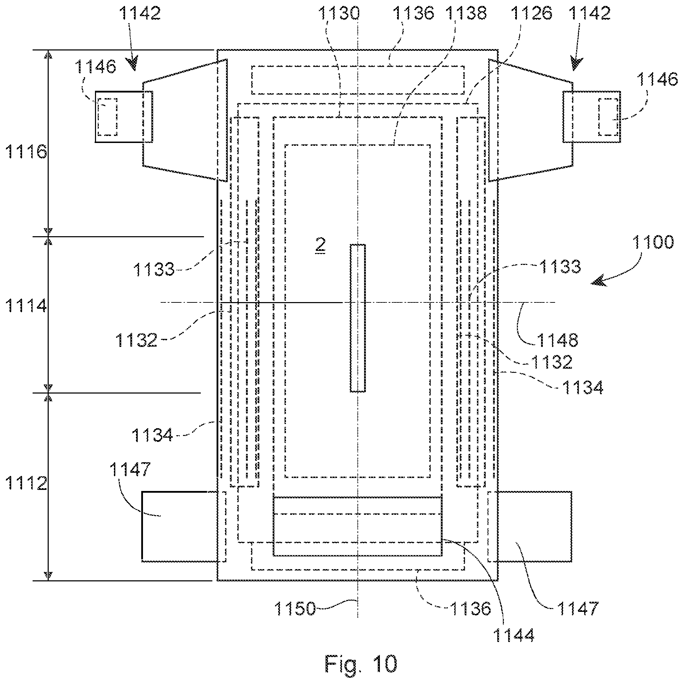

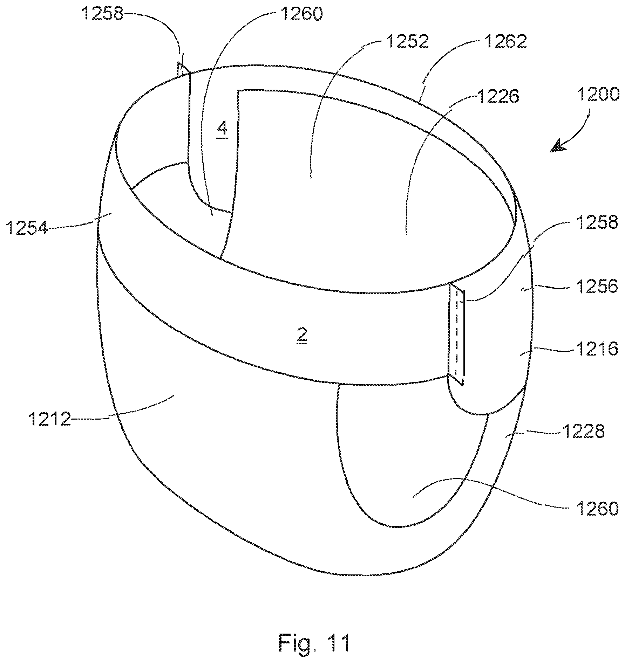

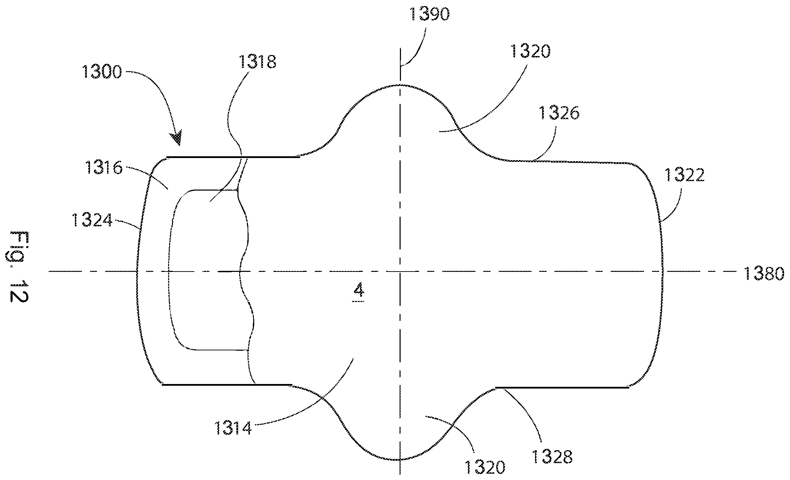

The flexible packages are made for hygiene articles 1004, in particular absorbent articles. The flexible package may enclose absorbent articles, such as a diaper, as shown in Fig. 10, an absorbent pant, as shown in Fig. 11, or a sanitary napkin, as shown in Fig. 12. The flexible package may enclose diapers.

Fig. 10 shows a plan view of an example diaper 1100, garment-facing surface facing the viewer in a flat, laid-out state (i.e., no elastic contraction). The diaper 1100 may comprise a front waist region 1112, a crotch region 1114, and a back waist region 1116. The crotch region 1114 may extend intermediate the front waist region 1112 and the back waist region 1116. The front wait region 1112, the crotch region 1114, and the back waist region 1116 may each be 1/3 of the length of the diaper 1110. The diaper 1110 may comprise a liquid permeable topsheet 1126, a liquid impermeable backsheet, and an absorbent core 1130 positioned at least partially intermediate the topsheet 1126 and the backsheet. The diaper 1110 may also comprise one or more pairs of barrier leg cuffs 1132 with or without elastics 1133, one or more pairs of leg elastics 1134, one or more elastic waistbands 1136, and/or one or more acquisition materials 1138. The acquisition material or materials 1138 may be positioned intermediate the topsheet 1126 and the absorbent core 1130. An outer cover material, such as a nonwoven material, may cover a garment-facing side of the backsheet. The diaper 1110 may comprise back ears 1142 in the back waist region 1116. The back ears 1142 may comprise fasteners 1146 and may extend from the back waist region 1116 of the diaper 1110 and attach (using the fasteners 1146) to the landing zone area or landing zone material 1144 on a garment-facing portion of the front waist region 1112 of the diaper 1110. The diaper 1110 may also have front ears 1147 in the front waist region 1112. The diaper 1110 may have a central lateral (or transverse) axis 1148 and a central longitudinal axis 1150. The central lateral axis 1148 extends perpendicular to the central longitudinal axis 1150.

Fig. 11 shows a front perspective view of an absorbent article in the form of a pant 1200. The pant 1200 may have a chassis 1252 (sometimes referred to as a central chassis or central panel) comprising a topsheet 1226, a backsheet 28, and an absorbent core 30 disposed at least partially intermediate the topsheet 26 and the backsheet 1228, and an optional acquisition material, similar to that as described above with respect to the diaper represented by Fig. 10. The pant 1200 may comprise a front belt 1254 in the front waist region 1212 and a back belt 1256 in the back waist region 1216. The chassis 1252 may be joined to a wearer-facing surface 4 of the front and back belts 1254, 1256 or to a garment-facing surface 2 of the belts 1254, 1256. Side edges of the front belt 1254 may be joined to side edges of the back belt 1256 to form two side seams 1258. The side seams 1258 may be any suitable seams known to those of skill in the art, such as butt seams or overlap seams, for example. When the side seams 1258 are permanently formed or refastenably closed, the pant 1200 in the form of a pant has two leg openings 1260 and a waist opening circumference 1262. The side seams 1258 may be permanently joined using adhesives or bonds, for example, or may be refastenably closed using hook and loop fasteners, for example.

Fig. 12 shows a plan view of an example sanitary napkin 1300, wearer-facing surface facing the viewer. The sanitary napkin 1300 may comprise a liquid permeable topsheet 1314, a liquid impermeable, or substantially liquid impermeable, backsheet 1316, and an absorbent core 1318. The liquid impermeable backsheet 1316 may or may not be vapor permeable. The absorbent core 1318 may have any or all of the features described herein with respect to the absorbent core1130 of diaper 1100 and, in some forms, may have a secondary topsheet 1319 (STS) instead of the acquisition materials disclosed above. The STS 1319 may comprise one or more channels. In some forms, channels in the STS 1319 may be aligned with channels in the absorbent core 1318. The sanitary napkin 1310 may also comprise wings 1320 extending outwardly with respect to a longitudinal axis 1380 of the sanitary napkin 1310. The sanitary napkin 1310 may also comprise a lateral axis 1390. The wings 1320 may be joined to the topsheet 1314, the backsheet 1316, and/or the absorbent core 1318. The sanitary napkin 1310 may also comprise a front edge 1322, a back edge 1324 longitudinally opposing the front edge 1322, a first side edge 1326, and a second side edge 1328 longitudinally opposing the first side edge 1326. The longitudinal axis 1380 may extend from a midpoint of the front edge 1322 to a midpoint of the back edge 1324. The lateral axis 1390 may extend from a midpoint of the first side edge 1328 to a midpoint of the second side edge 1328. The sanitary napkin 1310 may also be provided with additional features commonly found in sanitary napkins as is known in the art.

The packages of the present disclosure may comprise a plurality of compressed hygiene article 1004, e.g., compressed absorbent articles. For example, packages of the present disclosure may be used for accommodating diapers. Referring again to Fig. 9, the package 1000 defines an interior space 1002 in which a plurality of absorbent articles 1004 are situated. The plurality of absorbent articles 1004 may be arranged in one or more stacks 1006. The absorbent articles may be packed under compression so as to reduce the size of the packages, while still providing an adequate number of absorbent articles per package. By packaging the absorbent articles under compression, caregivers can easily handle and store the packages, while also providing distribution savings to manufacturers owing to the size of the packages.

As said above, the polymeric film 100 may comprise more than one layer. For example, the polymeric film 100 may comprise a first outer layer, a second outer layer and one or more inner layers.

The first outer layer may face towards the interior of the flexible package, with first areas of the first outer layer being sealed to second areas of the first outer layer to form seams. Such sealed areas may have been treated with a processing aid prior to sealing to help obtain a durable seam. Alternatively or in addition, the first outer layer may comprise certain additives to improve the seal strength.

The second outer layer may comprise a printed surface area. Similar to the first outer layer, the second outer layer may have been treated with a processing aid prior to printing to improve the printing quality. Alternatively or in addition, the second outer layer may comprise certain additives to improve the printing quality, in particular the second outer layer may comprise certain additives to increase whiteness and to reduce hues of other colors.

Generally, it may be desirable to have more of the PCR polymeric material in the one or more inner layers than in the first and/or second outer layer of the polymeric film.

The one or more inner layers, in total, may comprise more of the PCR polymeric material than each of the first and second outer layers. Hence, the total amount of PCR polymeric material in the one or more inner layers may be higher than the total amount of the PCR polymeric material in the outer layers.

The one or more inner layers may form at least 50 weight-% of the total basis weight of the polymeric film. The percentage of the PCR polymeric material in the one or more inner layers based on the total basis weight of the one or more inner layers (the combined basis weight if there is more than one inner layer) may be higher than the percentage of the PCR polymeric material in the first outer layer based on the total basis weight of the outer layer. Alternatively or in addition, the percentage of the PCR polymeric material in the one or more inner layers based on the total basis weight of the one or more inner layers (the combined basis weight if there is more than one inner layer) may be higher than the percentage of the PCR polymeric material in the second outer layer based on the total basis weight of the outer layer.

Despite comprising traces of the metal barrier layer of the laminate to be recycled, the polymeric film incorporating the PCR material may exhibit very low levels of defects.

One property associated with films comprising recycled material is the formation or presence of holes. A hole may comprise a visually discernible area of film where the absence film mass is apparent. Holes are not consumer or manufacturer desired in that products may not be sealed off from an external environment. Without wishing to be bound by theory, it is believed that contaminants brought in with recycled material and/or improper incorporation of recycled material into a newly-formed film may result in numerous holes. The polymeric film may have an Average Hole Count from 0.0 to 10.0, preferably from 0.0 to 8.0, more preferably from 0.0 to 5.0, or most preferably from 0.0 to 3.0 or even of 0.0, according to the Gel Count Test Method disclosed herein.

Another property associated with polymeric films comprising recycled material is the presence of gels. Without wishing to be bound by theory, it is believed that incorporation of increasing amounts of recycled material into a film may result in the formation of an increased number of gels in a finished film. Gels are not desired because they may distort printing of the films and/or appear to consumers as a defect.

It is also believed that the incorporation of increasing amounts of recycled material into a film may result in the formation of larger gels and/or gels that more prominently stand off from the surface of the film. These type of gels are not desired because they may distort printing of the films and/or appear to consumers as a defect. The polymeric film may have an Average Relative Gel Height from 0.0% to 150.0%, preferably from 0.0% to 125.0%, more preferably from 0.0% to 100.0%, still more preferably from 0.0% to 75.0%, or most preferably from 0.0% to 50.0%, according to the Gel Count Test Method disclosed herein.

Another property associated with films comprising recycled material is the presence of dark spots in the films or on the surface of the films. Dark spots are not desired because they may distort printing of the films or appear to consumers as a defect. Without wishing to be bound by theory, it is believed that the occurrence of dark spots may increase with an increasing amount of recycled material incorporated into a film due, at least in part, to increased contamination from the recycled material and/or recycled material receiving additional heat and processing steps. The polymeric film may have a Total Spot Count from 0.0 to 100.0, preferably from 0.0 to 50.0, more preferably from 0.0 to 25.0, or still more preferably from 0.0 to 5.0, according to the Gel Count Test Method disclosed herein.

An important property of the polymeric films and the flexible packages is the print quality. As already discussed, gel formation can lead to reduced print quality. The print quality may be measured by counting spots, which comprise no ink or reduced amounts of ink. In particular, in full tone areas of color, white spots may be counted by eyes sight. Specimen of full colored area of 50mm x 50mm are visually inspected for print defects. The size of the defects is determined as the widest distance within the defect by a ruler. The polymeric films may have less than 25, preferably less than 10, more preferably less than 5 or even no print defects with a size larger than 1 mm in a specimen of colored area of 50mm x 50mm. In addition or alternatively, the polymeric films may have less than 50, preferably less than 25, more preferably less than 10 or even less than 5 print defects with a size between 0.5 mm and 1 mm in a specimen of colored area of 50mm x 50mm. In addition or alternatively, the polymeric films may have less than 100, preferably less than 50, more preferably less than 30 or even less than 10 print defects with a size between 0.05 mm and 0.5 mm in a specimen of colored area of 50mm x 50mm. However, print defects may be present in the polymeric films and flexible packages due to remaining metal and/ or paper contaminations within the PCR material.

The polymeric films and flexible packages may exhibit no print defects resulting from loss of contact between the print plate and the polymeric film surface. Larger gels in the film may lead to such loss of contact between the print plate and the polymeric film surface. Such defects typically have a donut-like appearance.

The choice of printing ink may decrease the amount of print defects. It has been found that the surface tension and/ or the viscosity of the ink can positively impact the print quality. Gels may be covered when inks with a higher surface tension and/ or lower viscosity are used. The printing ink may have a surface tension of at least 32 dynes/cm, preferably at least 34 dynes/cm, more preferably at least 36 dynes/cm, even more preferably at least 38 dynes/cm or even at least 40 dynes/cm. In addition or alternatively, the printing ink may have a viscosity of at least 10 Centipoise or at most 300 cP.

The invention also relates to a use of post-consumer recycled (PCR) material for making flexible package made of polymeric film 100 for hygiene articles 1004. The PCR polymeric material is derived from recycling of a laminate comprising a paper layer, a metal barrier layer and a polymer layer. In particular the PCR polymeric material may be derived from recycling of a laminate comprising a paper layer, a metal barrier layer and a polymer layer comprised in paper composite cartons, in particular paper composite cartons used for beverages or other liquid consumer goods.

### Method of making a flexible package made of polymeric film for hygiene articles

The invention also relates to a method of making a flexible package made of polymeric film 100 for hygiene articles 1004. The method comprises the steps of
a) obtaining post-consumer recycled (PCR) polymeric material from post-consumer recycling of a laminate comprising a paper layer, a metal barrier layer and a polymer layer;
b) providing the PCR polymeric material obtained in step a);
c) melting the PCR polymeric material and optionally mixing it with further polymeric material different from the PCR polymeric material;
d) extruding the molten polymeric material to form a polymeric film;
e) optionally providing print on one or both surfaces of the polymeric film; and
f) converting the polymeric film into the flexible package.

Step a) may further comprise the steps of
a1) obtaining post-consumer paper composite cartons, in particular paper composite cartons used for beverages or other liquid consumer goods; and/ or
a2) defibering the paper composite cartons, in particular paper composite cartons used for beverages or other liquid consumer goods.
The paper composite cartons may be obtained by sorting post-consumer waste or by providing a specific waste stream. The paper composite cartons, in particular paper composite cartons used for beverages or other liquid consumer goods, may be defibered in paper mills.

Step a) may further comprise the steps of
a3) aqueous delamination of the laminate to separate paper, metal barrier layer and polymer layer from each other.

The delamination step a3) may include the step of shredding the laminate to maximize the contact surface. Additionally, the shredded laminate is subjected to an aqueous separation solution. Heat may be applied during the delamination step and the solution with the shredded laminate may be stirred. After separation of the laminate, the solution may be sieved to obtain paper, barrier layer and polymeric layer components. The paper, barrier layer and polymeric layer components may be washed using an aqueous washing solution, preferably water. The separation of paper, barrier layer and polymeric layer components may be done via conventional sorting procedures. The paper, barrier layer and polymeric layer components may be dried prior or subsequent to separation.

The metal, in particular aluminum, and /or paper obtained in steps a2) and/ or a3) may be recycled.

Step a) may further comprise the steps of
a4) melt filtration of the polymeric material obtained from the polymer layer;
a5) optionally adding a colorant to the filtrated polymeric material;
a6) optionally adding a compatibilizer to the filtrated polymeric material, in particular adding a compatibilizer to the filtrated polymeric material in an amount such that processability in blown extrusion and mechanical properties of the extruded polymeric film is improved;
a7) regranulating the polymeric material to obtain PCR polymeric resin pellets.

Via melt filtration the polymeric material obtained from the polymer layer may be further purified. In particular, barrier layer material such as metals, in particular aluminum, and other contaminants may be removed. Prior to regranulating the polymeric material to obtain PCR polymeric resin pellets, additives such as colorants and/ or compatibilizers may be added.

Step b) may further comprise the steps of
b2) providing post-industrial recycled (PIR) polymeric material,; and
b3) optionally providing virgin polymeric material, preferably providing at most 90 weight-%, preferably at most 70 weight-%, more preferably at most 50 weight-%, even more preferably at most 20 weight-%, even more preferably at most 15 weight-% or even preferably at most 10 weight-% of virgin polymeric material based on the total weight of the polymeric film; and the optional virgin polymeric material.

The PIR polymeric material and the PCR polymeric material and optionally the virgin polymeric material may be jointly molten in step c) and jointly extruded to form a polymeric film in step d).

The PCR polymeric material may be provided such that is constitutes at least 5 weight-%, preferably at least 10 weight-%, more preferably at least 15 weight-%, even more preferably at least 20 weight-%, even more preferably at least 25 weight-% or even at least 35 weight-% of PCR polymeric material based on the total weight of the polymeric film.

The PIR polymeric material may be provided such that it constitutes at least 30 weight-%, preferably at least 40 weight-%, more preferably at least 50 weight-%, even more preferably at least 60 weight-% or even at least 80% of PIR polymeric material based on the total weight of the polymeric film.

The virgin polymeric material may be provided such that is constitutes at most 90 weight-%, preferably at most 70 weight-%, more preferably at most 50 weight-%, even more preferably at most 20 weight-%, even more preferably at most 15 weight-% or even preferably at most 10 weight-% of virgin polymeric material based on the total weight of the polymeric film.

Extrusion of the molten polymeric material in method step d) may be done such as to form a polymeric monolayer film. Extrusion of the molten polymeric material in method step d) may be done such as to form a polymeric monolayer film having one or more inner layers, a first outer layer and a second outer layer, as is described below in more detail. The composition of the different layers may not be the same

Method step f), converting the polymeric film into the flexible package, can be done by any method known in the art. Forming flexible packages of polymeric films are widely known and a multitude of methods and numerous different configurations, shapes and sizes of flexible packages are available on the market.

The polymeric film formed in method step e) may comprise a first outer layer, a second outer layer and one or more inner layers. The first outer layer may face towards the interior of the flexible package wherein first areas of the first outer layer may be sealed to second areas of the first outer layer to form seams. The second outer layer may comprise a printed surface area.

### Flexible package and flexible package with wicket panel

The empty flexible packages may be configured as a wicketed package. As an example, such wicketed, flexible packages may comprise a first panel, a second panel, and a wicket panel. The first panel defines a first right side edge portion, a first left side edge portion, and a first bottom edge portion. The second panel defines a second right side edge portion, a second left side edge portion, a hooded portion, and a second bottom edge portion. Of course, flexible packages not comprising a hooded portion are similarly applicable and useful for the present invention. The wicket panel may be connected with the second bottom edge portion of the second panel. The first and second panels and combine to define an interior and an exterior of the package. A first seam joins the first right side edge portion of the first panel with the second right side edge portion of the second panel. A second seam joins the first left side edge portion of the first panel 102 with the second left side edge portion of the second panel.

The wicket panel comprises at least one wicket aperture, each wicket aperture configured to receive a wicket. The wicket panel may include two wicket apertures. The first bottom edge portion and the second bottom edge portion of the first and second panels and combine to define a second opening in the package. The wicket panel may extend beyond the first bottom edge portion of the first panel such that the wicket panel does not overlap the first panel.

A plurality of empty (i.e. not filled with absorbent articles or any other products) wicketed, flexible packages may be stacked one on top of the other such that the wicket apertures of each wicketed, flexible package are aligned. A wicket may extend through the wicket apertures of each wicketed, flexible package to hold the stack of wicketed, flexible packages together. In an exemplary configuration wherein wicketed, flexible packages in a stack each comprise two wicket apertures, two wickets may be used. Various types of wickets for holding a stack of wicketed, flexible packages together may be used. The plurality of wicketed, flexible packages are held together by the wickets while the absorbent articles are introduced into the wicketed, flexible package through the second opening. Once the wicketed, flexible package is filled e.g. with absorbent articles, the first and second panels and may be sealed together and the wicket panel may cut away from the second panel. The removed wicket panel may form the precursor polymeric film material used in the present invention.

The method of converting the polymeric film into a flexible package may be performed by any method known in the art, dependent which configuration, shape and size the flexible package is intended to take. For example, if converting the polymeric film into a wicketed, flexible packages, the method may include seaming first right and left side edge portions of a first panel, a second panel, and a gusset. A first seam may be formed in the first and second right side edge portions of the first and second panel and and the right side edge portion of the gusset respectively. A second seam may be formed in the first and second left side edge portions of the first and second panel and and the left side edge portion of the gusset, respectively. The first and second seams and may be formed in various ways, including ultrasonic welding, hot air seaming, adhesives, and the like. The steps of cutting the continuous length of material to form discrete wicketed bags and forming the first and/or second seams and may occur concurrently. Alternatively, the steps of cutting and seaming may occur sequentially.

A second opening is defined by the second end portions of the first and second panels and. The wicketed, flexible packages may be filled with hygiene articles 1004, in particular absorbent articles by inserting the hygiene articles 1004, in particular absorbent articles through the second opening.

Once the wicketed, flexible package is filled with hygiene articles 1004, in particular absorbent articles, the second end portions of the first and second panels and may be joined together to form seam. Like the first and second seams and, the third seam may be formed in various ways as described above.

Additionally, the wicket panel may be cut away from the second panel to form the flexible package. The flexible package may comprise an opening feature, such as perforation. The steps of forming the third seam and cutting the wicket panel may occur concurrently. Alternatively, the steps of forming the third seam and cutting the wicket panel may occur sequentially. Various methods may be used to cut the wicket panel from the second panel. Exemplary cutting apparatuses include knife rolls, die cutters, and lasers.

### Examples

PCR polymeric material was derived from post-consumer recycling of paper composite cartons used for beverages or other liquid consumer goods. The paper composite cartons were subjected to aqueous delamination of the laminate to separate paper, aluminum barrier layer and polyethylene polymer layer from each other. The polyethylene polymeric material was subjected to melt filtration. Two different PCR polyethylene polymeric materials were obtained, one with a melt flow rate of 4.5 and one with a lower melt flow rate of between 1.0 and 2.0. The PCR polyethylene polymeric material with a melt flow rate of between 4.0 and 5.0 g/ 10min comprised 356 µg/g Aluminum according to the Metal Content Test.

The PCR polyethylene polymeric material with a melt flow rate of between 4.0 and 5.0 g/ 10min was subjected to the Trace Chemicals Test Method.

**Table 1: Trace Chemical content of PCR polyethylene polymeric material with a melt flow rate of between 4.0 and 5.0 g/ 10min**

| **Substance** | **Amount** |
|---|---|
| PCB 77 | 91.6 ng/kg |
| PCB 118 | 828 ng/kg |
| OCDD | 89.4 ng/kg |
| OCDF | 3.66 ng/kg |
| Bisphenol A | 320 µg/kg |
| diisononyl phthalate | 17000 µg/kg |

The PCR polyethylene polymeric material with a melt flow rate of between 4.0 and 5.0 g/ 10min comprised low levels of trace chemicals.

Examples 1, 2 and 3 are extruded three-layer films with a middle bulk layer and two outer layers with the middle layer forming 50% by weight of the total basis weight of the polymeric films and each outer layer 25% by weight respectively. The layers had a total target thickness of 70 ± 3 µm. The PCR polymeric materials were incorporated in the middle bulk layer. The outer layers contained PIR and virgin PE polymeric materials.

Example 1 contained a middle layer with 20 % by weight of the middle layer PCR material with a melt flow rate of 4-5 g/ 10min. In total, Example 1 contained 10% PCR PE derived from post-consumer recycling of paper composite cartons used for beverages or other liquid consumer goods, 60% PIR PE and 30 % virgin PE.

Example 2 contained a middle layer with 20 % by weight of the middle layer PCR material with a melt flow rate of 4-5 g/ 10min. In total, Example 2 contained 10% PCR PE derived from post-consumer recycling of paper composite cartons used for beverages or other liquid consumer goods, 40% PIR PE and 50 % virgin PE.

Example 3 contained a middle layer with 20 % by weight of the middle layer PCR material with a melt flow rate of between 1.0 and 2.0 g/ 10min. In total, Example 2 contained 10% PCR PE derived from post-consumer recycling of paper composite cartons used for beverages or other liquid consumer goods, 40% PIR PE and 50 % virgin PE.

For each of Examples 1-3, 30 film specimen were extruded and the following mechanical properties of polymeric films measured and averaged over the 30 specimen according to the Mechanical Properties Test Method: tensile strength at cross-direction break, elongation at cross-direction break, tensile strength at machine-direction break and elongation at machine-direction break.

**Table 2: Mechanical properties of Examples 1-3**

| **Example** | **Tensile Strength CD break [N/mm]** | **Elongation CD break [%]** | **Tensile Strength MD break [N/mm]** | **Elongation MD break [%]** |
|---|---|---|---|---|
| 1 | 1.51 | 797 | 1.38 | 560 |
| 2 | 1.80 | 900 | 1.64 | 685 |
| 3 | 1.73 | 877 | 1.46 | 632 |

All three examples exhibited good mechanical properties despite the relatively high amount of total recycled material contained and the aluminum and paper contamination in the PCR material.

Example 2 was chosen to be printed and to produce more than 2000 flexible packages. While comprising visually detectable print defects due to gel formation due to residing aluminum and/ or paper in the film, the printed films exhibited no print defects with a size larger than 1 mm in the printed area. The obtained flexible packages exhibited no defects (holes, sealing defects etc.).

### TEST METHODS

### Mechanical Properties

The mechanical properties of polymeric films may be measured.

Side seam tensile strength may be measured by taking a sample from a flexible packaging formed by the polymeric film and cutting out a sample which comprised the side seam. In the side seam, two layers of the polymeric film were sealed to each other (= "2-ply").

Thickness of the film was measured in accordance with ISO 4593:1993. The mechanical properties were measured in accordance with ISO 527-1:2012.

### Tensile strength, tensile strength at 10% elongation, and elongation at break:

Load cell force range: Measured force must be within 10% and 90 % of range
Signal sampling frequency: 50 Hz
Load cell: 5000 N; Accuracy: +/- 0.5 %
Sample dimensions: 25.4mm width (1 inch); Sample length: 150mm
Crosshead speed (= speed of elongation): 127 mm/min
The tests were carried out at 22°C and 55% RH.

### Gel Count Test Method

The Gel Count Test Method is used to quantify the incidence of defects of interest in sample packaging film of interest. Evaluation on several specimens of a sample packaging film is primarily done visually, and several output parameters relating to gels, dark spots, and holes are established.

Five like specimens, rectangular and 20 cm × 20 cm, are taken from a sample packaging film of interest. If the film is available as roll stock, the five specimens are cut from one or more rolls of material, with material preferentially being taken from unprinted regions. If the packaging film of interest is not available in roll stock form and is, for example, only available as formed packaging bags for containing hygiene articles, these five specimens are extracted from one or more packaging bags. The surface of the film facing inward in the bag (i.e., toward and generally enveloping hygiene articles when the bag is loaded with product) is defined as the inner surface of the film and the surface of the film facing outward away from hygiene articles is defined as the outer surface of the film. In the case the specimens are taken from roll stock before bags are formed (and potentially before any printed step present in the film conversion process), if the surface intended to be an outer surface after conversion is known, this is considered to be the outer surface and the opposite surface is considered to be the inner surface. If the surface intended to be an outer surface is unknown, the outer surface is determined in the dark-spot-counting procedure as described below.

Each of the five film specimens is backlit by being placed on a white light box sequentially and viewed with the naked eye. For each of the five film specimens, any holes or tears apparent in the film are identified and marked. A hole may comprise a visually discernible area of film where the absence film mass is apparent. A tear may comprise a hole which has an elongated opening or absence of film mass extending in one direction. Any gels apparent are also identified and marked. (For the purposes of this method, a gel is a distinct individual entity, generally visually discernible and consistent with a small clump of cross-linked polymer resin or as defined elsewhere within this patent document.) A caliper gauge with flat, round foot 6.00 to 6.25 mm in diameter and exerting less than 3.5 N of force is used to measure and record the absolute height of each gel to the nearest 0.01 mm. One example of a suitable apparatus is caliper gauge model ID-C112BS available from Mitutoyo Corporation, Kawasaki, Japan, or equivalent. For each gel, the caliper of proximal, well-formed film is also measured in like fashion. For each gel, its gel height is defined as $gel height \left[mm\right] = absolute height of gel \left[mm\right] - proximal film caliper \left[mm\right]$ and its relative gel height, in percent, is defined as $relative gel height \left[%\right] = \frac{gel height \left[mm\right]}{proximal film caliper \left[mm\right]} \times 100 % .$

For each gel, the gel height and relative gel height are measured, calculated, and recorded. If a cluster of gels containing individually discernible gels spaced so closely such that the caliper gauge cannot measure the height of each gel individually, the gel height and relative gel height of the tallest gel in the cluster is assigned to all gels in the cluster.

Each surface of each of the five film specimens is then viewed by the naked eye front-lit under diffuse white light, and any dark specs or spots are identified and counted. For specimens of sample film for which the inner surface and outer surface are not known, the surface with the greater number of spots is considered the inner surface. For specimens of sample film for which the inner surface and outer surface is known, the number of dark specs or spots are counted as appearing on the inner surface or the outer surface depending which is being viewed.

For the sample film evaluated via the five specimens, the Average Hole Count is defined as the total number of holes observed among the five specimens divided by five and is reported to the nearest tenth. The Average Gel Count is defined as the total number of gels observed among the five specimens divided by five and reported to the nearest tenth. The Average Gel Height is the arithmetic mean among all gel heights recorded, reported to the nearest 0.002 mm. The Average Relative Gel Height is the arithmetic mean among all relative gel heights recorded and is reported to the nearest 0.1%. The Average Inner-Surface Spot Count is the total number of dark specs or spots observed on the inner surface of the five specimens divided by five and reported to the nearest tenth. The Average Outer-Surface Spot Count is the total number of dark specs or spots observed on the outer surface of the five specimens divided by five and reported to the nearest tenth. The Average Total Spot Count is the sum of the Average Inner-Surface Spot Count and the Average Outer-Surface Spot Count and is reported to the nearest tenth.

### Trace Chemicals Test Method

The content on a mass per mass basis of trace chemicals present in packaging film or recycled polymeric material is determined using methods containing sample extraction and instrumental analysis approaches known of those of skill in the art and understood to be capable of extracting and detecting essentially all of a particular trace chemical in polymeric film or the PCR polymeric material. Suitable overall method specifics vary based on the particular trace chemical being quantified, though a common overall method structure is generally followed. A suitable method generally begins with increasing the surface area of a representative packaging film or recycled polymeric material sample by cutting, shredding, and/or milling into small particles. An extraction step is then performed using a solvent capable of dissolving or diffusing into the polymer sample and in which the trace chemical is readily soluble. The extraction process conditions (e.g., time and temperature) are optimized to maximize trace chemical recovery and common approaches include but are not limited to Soxhlet extraction, accelerated solvent extraction, and/or ultrasonic extraction. Internal standards (usually stable-isotope-labeled versions of the trace chemicals if mass spectrometry-based detection approaches are used) are added during the extraction step to account for potential losses during extraction and subsequent processing steps. After extraction is complete, the extraction solvent is isolated and worked up for instrumental analysis. Depending on the solvent used for extraction, the required quantitation limit, and the specific instrumental approach used, generation of a specimen suitable for instrumental analysis may require multiple cleanup and/or concentration steps including but not limited to centrifugation/filtration, liquid/liquid extraction, and/or column chromatography. The trace chemical in the worked-up specimen is then quantified using instrumental methods (e.g., GC-MS, GC-MS/MS, GC-HRMS, LC-MS/MS, etc.) known to those of skill in the art and specifically adapted to the trace chemical of interest. Suitable instrumental approaches may be developed de novo or may be based on standard, known methods for particular trace chemicals (e.g., US EPA Method 1613 in the case of dioxins and furans or US EPA 1668 for PCBs). Based on a combination of raw instrumental quantitative results, extraction solvent workup steps, original extraction volume, and starting specimen mass, the mass content of a trace chemical per mass of starting specimen material is calculated and is reported as the level of the trace chemical in the packaging film tested. Given the generally low levels of trace chemicals present, common units used in reported levels of trace chemicals are µg/kg and ng/kg (equivalent to the ppb and ppt levels, respectively).

One of skill in the art will recognize that trace chemicals are often present at very low "trace" levels, meaning that under some circumstances, environmental and/or laboratory background levels of these same trace chemicals may give rise to false positive detects or falsely elevated results if appropriate precautions are not taken. While one of skill in the art recognizes that a suite of measures may be needed to minimize such false results, one obligatory measure is to include a true "blank" beginning with solvent carried in parallel through the extraction step, extraction solvent workup, and instrumental analysis.

Using the suitable overall methodology outlined above, one of skill in the art may quantify a range of trace chemicals potentially present in a packaging film or recycled polymeric material sample of interest. For example, the level of PCB 77 (CAS RN 32598-13-3) may be determined and reported as mass of trace chemical per mass of polymeric film or the PCR polymeric material in units of ng/kg. Similarly, the level of PCB 118 (CAS RN 31508-00-6) may be determined and reported in units of ng/kg, the level of OCDD (CAS RN 3268-87-9), a dioxin, may be determined and reported in units of ng/kg, the level of OCDF (CAS RN 39001-02-0), a furan, may be determined and reported in units of ng/kg, the level of bisphenol A (CAS RN 80-05-7), also known as BPA, may be determined and reported in units of µg/kg, and the level of diisononyl phthalate (CAN RN 28553-12-0), also known as DINP, may be determined and reported in units of µg/kg.

### Metal Content Test

The metal content of the film is determined as follows:

### Test Sample Preparation - Total Microwave Digestion

Approximately 0.1 g of sample was transferred and weighed into a pre-cleaned 15 mL TFM UltraWAVE tube (Milestone Srl, Bergamo, Italy). Each sample was combined with 4 mL nitric acid, 1 mL hydrochloric acid and 2 mL fluoroboric acid. Samples were then digested using an UltraWAVE Microwave Digestion System heating to a final temperature of 260 °C. After digestion/dissolution, samples were then transferred to 50 mL polypropylene tubes, combined with internal standard, and diluted to 50 mL with deionized water. The resulting test solutions were visually checked to confirm full digestion of the sample such that the solution yielded a transparent solution with no fine particulates present. The transparent solutions were analyzed by ICP-MS/MS for the metal content, in particular for aluminum (Al).

### Calibration Curve Preparation

Working standards were prepared by combining appropriately diluted reference standards (Inorganic Ventures, Christiansburg, Virginia, USA), covering the concentration ranges of interest (e.g. 10-2000 ng/mL Al), together with selected internal standard (Sc). The final composition of these working standards were matched in acid concentration to the prepared samples for matrix-matching purposes. Results from analysis of these working standards yielded the requisite data to prepare calibration curves for quantitation metal content, in particular for aluminum (Al).

### ICP-MS/MS Analysis

Prepared standard and test samples were analyzed using an ICP-MS/MS such as an Agilent 8800 ICP QQQ (Agilent Technologies, Inc. Santa Clara, CA, USA) using multiple detection schemes optimized for metals, in particular aluminum. Instrumentation and detection schemes were optimized according to manufacturer recommendations. One detection scheme was chosen for reporting. The reported detection scheme was 27 → 27 Al [He]. Selectivity for the reported detection scheme was enhanced by employing a collision gas (helium).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. Flexible package made of polymeric film for hygiene articles, wherein the polymeric film comprises post-consumer recycled (PCR) polymeric material derived from recycling of a laminate comprising a paper layer, a barrier layer and a polymer layer.

2. Package according to any one of the preceding claims, wherein the PCR polymeric material is derived from post-consumer recycling of paper composite cartons, in particular paper composite cartons used for beverages or other liquid consumer goods.

3. Package according to any one of the preceding claims, wherein the polymeric film comprises at least 5 weight-%, preferably at least 10 weight-%, more preferably at least 15 weight-%, even more preferably at least 20 weight-%, even more preferably at least 25 weight-% or even at least 35 weight-% of PCR polymeric material based on the total weight of the polymeric film.

4. Package according to any one of the preceding claims, wherein the polymeric film additionally comprises post-industrial recycled (PIR) polymeric material, preferably comprises at least 30 weight-%, preferably at least 40 weight-%, more preferably at least 50 weight-%, even more preferably at least 60 weight-% or even at least 80% of PIR polymeric material based on the total weight of the polymeric film.

5. Package according to any one of the preceding claims, wherein the polymeric film comprises at most 90 weight-%, preferably at most 70 weight-%, more preferably at most 50 weight-%, even more preferably at most 20 weight-%, even more preferably at most 15 weight-% or even preferably at most 10 weight-% of virgin polymeric material based on the total weight of the polymeric film or even is devoid of virgin polymeric material.

6. Package according to any one of the preceding claims, wherein the polymeric film comprises polyolefin, preferably polyethylene (PE), more preferably low density polyethylene (LDPE).

7. Package according to any one of the preceding claims, wherein the barrier layer comprised by the laminate to be recycled comprises aluminum, preferably the barrier layer comprised by the laminate to be recycled is an aluminum barrier layer.

8. Package according to claim 7, wherein the polymeric film comprises from 1 µg to 1500 µg, preferably from 5 µg to 1000 µg, more preferably from 10 µg to 800 µg, even more preferably from 15 µg to 500 µg, even more preferably from 20 µg to 300 µg, or even from 25 µg to 100 µg aluminum per 1 g of the polymeric film according to Metal Content Test.

9. Package according to any one of the preceding claims, wherein the polymeric film meets one or more of the criteria a) to f) by comprising less than
a) 40 ng/kg, preferably less than 10 ng/kg of PCB 77;
b) 400 ng/kg, preferably less than 100 ng/kg of PCB 118;
c) 30 ng/kg, preferably less than 10 ng/kg of OCDD;
d) 2.0 ng/kg, preferably less than 0.5 ng/kg of OCDF;
e) 100 µg/kg, preferably less than 40 µg/kg of bisphenol A;
and/ or
f) 5,000 µg/kg, preferably less than 2,000 µg/kg of diisononyl phthalate; according to the Trace Chemicals Test Method described herein.

10. Package according to any one of the preceding claims, wherein the flexible package encloses absorbent articles.

11. Use of post-consumer recycled (PCR) material for making flexible package made of polymeric film for hygiene articles, wherein the PCR polymeric material is derived from recycling of a laminate comprising a paper layer, a metal barrier layer and a polymer layer, in particular from recycling of a laminate comprising a paper layer, a metal barrier layer and a polymer layer comprised in paper composite cartons, in particular paper composite cartons used for beverages or other liquid consumer goods.

12. Method of making a flexible package made of polymeric film for hygiene articles, the method comprising the steps of
a) obtaining post-consumer recycled (PCR) polymeric material from post-consumer recycling of a laminate comprising a paper layer, a metal barrier layer and a polymer layer;
b) providing the PCR polymeric material obtained in step a);
c) melting the PCR polymeric material and optionally mixing it with further polymeric material different from the PCR polymeric material;
d) extruding the molten polymeric material to form a polymeric film;
e) optionally providing print on one or both surfaces of the polymeric film; and
f) converting the polymeric film into the flexible package.

13. The method of claim 12, wherein step a) further comprises the steps of
a1) obtaining post-consumer paper composite cartons, in particular paper composite cartons used for beverages or other liquid consumer goods;
a2) defibering the paper composite cartons, in particular paper composite cartons used for beverages or other liquid consumer goods.

14. The method of claims 12 or 13, wherein step a) further comprises the step of
a3) aqueous delamination of the laminate to separate paper, metal barrier layer and polymer layer from each other.

15. The method of any one of claims 12 to 14, wherein step a) further comprises the steps of
a4) melt filtration of the polymeric material obtained from the polymer layer;
a5) optionally adding a colorant to the filtrated polymeric material;
a6) optionally adding a compatibilizer to the filtrated polymeric material, in particular adding a compatibilizer to the filtrated polymeric material in an amount such that processability in blown extrusion is improved;
a7) regranulating the polymeric material to obtain PCR polymeric resin pellets.

16. The method of any one of claims 12 to 15, wherein step b) further comprises the steps of
b2) providing post-industrial recycled (PIR) polymeric material, preferably providing at least 50 weight-%, preferably at least 60 weight-%, more preferably at least 70 weight-%, even more preferably at least 80 weight-% or even at least 90% of PIR polymeric material based on the total weight of the polymeric film; and
b3) optionally providing virgin polymeric material, preferably providing at most 90 weight-%, preferably at most 70 weight-%, more preferably at most 50 weight-%, even more preferably at most 20 weight-%, even more preferably at most 15 weight-% or even preferably at most 10 weight-% of virgin polymeric material based on the total weight of the polymeric film;
and the optional virgin polymeric material, the PIR polymeric material and the PCR polymeric material are jointly molten in step c) and jointly extruded to form a polymeric film in step d).
